# EUROPEAN PATENT APPLICATION

(11) **EP 4 851 627 A1**
(43) Date of publication of application: **09.12.2026**
(21) Application number: 25748181.2
(22) Date of filing: 28.01.2025
(51) Int. Cl.: G01N 33/543, G01N 33/531, G01N 33/545

(54) **IMMUNOREACTION REAGENT, METHOD FOR PRODUCING SAME, AND IMMUNOREACTION REAGENT KIT**

(30) Priority: 30.01.2024 JP 2024011489; 30.01.2024 JP 2024011490; 30.01.2024 JP 2024011491; 05.12.2024 JP 2024212245; 05.12.2024 JP 2024212244; 05.12.2024 JP 2024212243; 26.12.2024 JP 2024229594; 26.12.2024 JP 2024229595; 26.12.2024 JP 2024229593
(71) Applicant: Tosoh Corporation, Shunan-shi, Yamaguchi 746-8501 (JP)
(72) Inventor: HATA, Tomoki, Ayase-shi, Kanagawa 252-1123 (JP); IIMURA, Naoki, Ayase-shi, Kanagawa 252-1123 (JP)
(74) Representative: Novagraaf International SA
(86) International application number: PCT/JP2025/002555
(87) International publication number: WO 2025/164600

(57) **Abstract**

The present invention addresses the problem of providing an immunoreaction reagent in a freeze-dried state, the reagent being used in an extracorporeal diagnostic agent or the like and having good measurement reproducibility. The problem is solved by obtaining an immunoreaction reagent by: letting solid-phase antibodies for antigens subject to measurement coexist with a defoaming agent in a solution and freeze-drying the solution; letting solid-phase antigens for antibodies subject to measurement coexist with a defoaming agent in a solution and freeze-drying the solution; or letting solid-phase antigens acting as competitors with antigens subject to measurement in bonding with antibodies for the antigens subject to measurement coexist with a defoaming agent in a solution and freeze-drying the solution.

## Description

### FIELD

The present invention relates to an immunoreaction reagent in a lyophilized state used for an in vitro diagnostic reagent and the like, a production method for the reagent, and an immunoreaction reagent kit.

### BACKGROUND

An antibody immobilized on a solid-phase support such as beads or microparticles (hereinafter referred to as "solid-phase antibody"), an antigen immobilized on a solid-phase support (hereinafter referred to as "solid-phase antigen"), an antibody to which a label such as an enzyme is bound (hereinafter referred to as "labeled antibody"), and an antigen to which a label such as an enzyme is bound (hereinafter referred to as "labeled antigen") have been widely used in fields such as clinical testing. However, since antibodies, antigens, enzymes, and the like are easily affected by temperature in terms of their activities, they cannot be stored for a long period in a state where the activities are maintained. For this reason, various stabilization methods have been proposed in order to maintain these activities.

PTL 1 reports that a labeled antibody is stabilized by adding a nonreducing sugar of a disaccharide or higher, a sugar alcohol, or the like, and then performing lyophilization. PTL 2 reports that human-derived alkaline phosphatase is stabilized by performing lyophilization in the presence of a sugar selected from the group consisting of galactose, lactose, and fructose, and albumin or dextran. However, even in cases where an appropriate compound, sugar, protein, or the like has been selected for an immunoreaction reagent in a lyophilized state so that the storage stability required in the field of clinical testing can be obtained, the reagent is often in the form of a hard dry cake, and has poor solubility after redissolution. As a result, measurement reproducibility becomes poor, and this has posed a problem in that highly accurate measurement of a component to be measured is prevented.

### [CITATION LIST]

### [PATENT LITERATURE]

[PTL 1] Japanese Unexamined Patent Publication (Kokai) No. H60-149972
[PTL 2] Japanese Patent Publication No. 4169344

### SUMMARY

### [TECHNICAL PROBLEM]

In the field of clinical testing, long-term storage stability is required for various reagents. Reproducibility at the time of measurement is also required. A reagent in a lyophilized state needs to be dissolved with a dissolving solution or the like when the reagent is used for measurement, but air bubbles may be generated during the dissolution due to pores (voids) derived by lyophilization. The present inventors found that there is variation in whether the air bubbles are generated and in the size of the air bubbles, and that the variation in whether the air bubbles are generated and in the size of the air bubbles causes deterioration of reproducibility. Accordingly, an object of the present invention is to provide an immunoreaction reagent in a lyophilized state that is used for in vitro diagnostic agents and the like and that shows favorable measurement reproducibility, a production method for the reagent, and an immunoreaction reagent kit.

### [SOLUTION TO PROBLEM]

As a result of intensive study to solve the above-described problems, the present inventors found that measurement reproducibility can be improved by adding an antifoaming agent to an immunoreaction reagent in a lyophilized state, thereby completing the present invention.

Specifically, the present invention is as follows.
(1) An immunoreaction reagent comprising:
   a lyophilized reagent containing a solid-phase antibody against an antigen to be measured, and an antifoaming agent.
(2) An immunoreaction reagent comprising:
   a lyophilized reagent containing a solid-phase antigen against an antibody to be measured, and an antifoaming agent.
(3) An immunoreaction reagent comprising:
   a lyophilized reagent containing a solid-phase antigen that competes with an antigen to be measured for binding to an antibody against the antigen to be measured, and an antifoaming agent.
(4) The immunoreaction reagent according to any one of (1) to (3), wherein the antifoaming agent is an emulsion-type water-soluble silicone-based antifoaming agent and/or a self-emulsifying water-soluble silicone-based antifoaming agent.
(5) A method of producing an immunoreaction reagent, the method comprising: allowing a solid-phase antibody against an antigen to be measured and an antifoaming agent to coexist in a solution; and lyophilizing the solution.
(6) A method of producing an immunoreaction reagent, the method comprising: allowing a solid-phase antigen against an antibody to be measured and an antifoaming agent to coexist in a solution; and lyophilizing the solution.
(7) A method of producing an immunoreaction reagent, the method comprising:
   allowing a solid-phase antigen and an antifoaming agent to coexist in a solution, the solid-phase antigen competing with an antigen to be measured for binding to an antibody against the antigen to be measured; and
   lyophilizing the solution.
(8) The method of producing an immunoreaction reagent according to any one of (5) to (7), wherein the antifoaming agent is an emulsion-type water-soluble silicone-based antifoaming agent and/or a self-emulsifying water-soluble silicone-based antifoaming agent.
(9) The method of producing an immunoreaction reagent according to any one of (5) to (8), wherein the solid-phase antibody or the solid-phase antigen is dispersed to prepare a suspension, and then the suspension is immediately frozen and lyophilized.
(10) An immunoreaction reagent kit comprising:
   a lyophilized reagent containing a solid-phase antibody against an antigen to be measured, and an antifoaming agent; and
   a reagent containing a labeled antibody capable of binding to the antigen.
(11) An immunoreaction reagent kit comprising:
   a lyophilized reagent containing a solid-phase antibody against an antigen to be measured, and an antifoaming agent; and
   a reagent containing a labeled antigen capable of binding to the solid-phase antibody.
(12) An immunoreaction reagent kit comprising:
   a lyophilized reagent containing a solid-phase antigen against an antibody to be measured, and an antifoaming agent; and
   a reagent containing a labeled antibody capable of binding to the antigen.
(13) An immunoreaction reagent kit comprising:
   a lyophilized reagent containing a solid-phase antigen and an antifoaming agent, the solid-phase antigen competing with an antigen to be measured for binding to an antibody against the antigen to be measured; and
   a reagent containing a labeled antibody capable of binding to the solid-phase antigen and the antigen to be measured.
(14) The immunoreaction reagent kit according to any one of (10) to (13), wherein the antifoaming agent is an emulsion-type water-soluble silicone-based antifoaming agent and/or a self-emulsifying water-soluble silicone-based antifoaming agent.

The present invention is described below in detail.

In the present invention, an antigen or an antibody is measured. In cases where an antigen is measured, examples of the antigen include, but are not limited to, substances used in clinical testing, such as squamous cell carcinoma-related antigen (SCC antigen), prostate-specific antigen (PSA), thyroid-stimulating hormone (TSH), thyroxine, triiodothyronine, testosterone, estradiol, progesterone, cortisol, aldosterone, homocysteine, β2-microglobulin (BMG), brain natriuretic peptide (BNP), and C-peptide. Among these, aldosterone and homocysteine are preferred. The reason is as follows. The present inventors have found that these antigens, in particular, are, although rarely, affected by interference from air bubbles in the measurement concentration range, resulting in variation in measured values. The present invention can solve such a phenomenon. On the other hand, in cases where an antibody is to be measured, examples of the antibody include, but are not limited to, substances used in clinical testing, such as human immunoglobulin, anti-thyroid peroxidase antibody, anti-thyroglobulin antibody, and TSH receptor antibody.

In the present invention, the solid-phase antibody refers to a solid-phase antibody that is obtained by immobilizing an antibody against the above-described antigen to be measured on a solid-phase support such as beads or microparticles, and that is insoluble in a solution in which an antigen-antibody reaction is carried out. The antibody does not necessarily need to be directly bound to the solid phase, and may be bound, for example, via avidin-biotin binding. Specifically, the antibody also includes a soluble antibody to which biotin is bound for binding to an avidin-solid phase complex.

In the present invention, the solid-phase antigen refers to a solid-phase antigen that is obtained by immobilizing an antigen against the above-described antibody to be measured on a solid-phase support such as beads or microparticles, and that is insoluble in a solution in which an antigen-antibody reaction is carried out. The antigen does not necessarily need to be directly bound to the solid phase, and may be bound, for example, via avidin-biotin binding. Specifically, the antigen also includes a soluble antigen to which biotin is bound for binding to an avidin-solid phase complex.

The labeled antibody refers to a labeled antibody that is obtained by binding a label to an antibody against the above-described antigen or antibody to be measured, and that is soluble in a solution in which an antigen-antibody reaction is carried out. The label does not necessarily need to be directly bound to the antibody, and may be bound, for example, via avidin-biotin binding. Specifically, the antibody also includes a soluble antibody to which biotin is bound for binding to an avidin-label complex.

The labeled antigen refers to a labeled antigen that is obtained by binding a label to the above-described antigen to be measured, and that is soluble in a solution in which an antigen-antibody reaction is carried out. The label does not necessarily need to be directly bound to the antigen, and may be bound, for example, via avidin-biotin binding. Specifically, the antigen also includes a soluble antigen to which biotin is bound for binding to an avidin-label complex.

As the solid phase, beads, microparticles, or wells can be used. Microparticles are particularly preferred. The solid phase may be an inorganic substance such as glass, metal, or ceramics, or may be an organic substance such as a high-molecular-weight polymer. Those beads and microparticles may contain a magnetic substance. The particle size of the microparticles is preferably 0.1 to 50 µm, more preferably 1 to 10 µm. The particle size of the beads is preferably 0.1 to 5 mm, more preferably 0.5 to 2 mm.

Examples of the label include, but are not limited to, an enzyme and a radioisotope. An enzyme is especially preferred, and examples of the enzyme include alkaline phosphatase and peroxidase.

In cases where an enzyme is used as the label, a substrate that reacts with the enzyme can be used. As the substrate, a fluorescent substrate, a chemiluminescent substrate, a bioluminescent substrate, a chromogenic substrate, or the like can be used.

The immunoassays using these are solid-phase methods, and include sandwich methods and competitive methods. Further, the antibody or antigen does not necessarily need to be directly bound to the solid phase, and the immunoassays include assays in which an antigen-antibody reaction is followed by a reaction step of binding the antibody or antigen to the solid phase, for example, assays in which an immunoreaction step by a sandwich method using a biotinylated antigen is followed by reaction with an avidin-solid-phase complex. Further, the antibody does not necessarily need to be directly bound to the label, and the immunoassays include assays in which an antigen-antibody reaction is followed by a reaction step of binding the label, for example, assays in which an immunoreaction step by a sandwich method using a biotinylated antibody is followed by reaction with an avidin-label complex.

The antibody in the present invention may be either a polyclonal antibody or a monoclonal antibody. An antibody derived from any animal species that produces an antibody may be used, and examples of the animal species include rabbits, goats, sheep, pigs, horses, mice, and rats. The form of the antibody may be a complete antibody, or may be an antibody fragment such as F(ab')₂ or Fab' obtained by cleaving the complete antibody by enzymatic treatment or chemical treatment. Further, scFv (single-chain variable fragment) and VHH antibodies can also be used.

The antigen competitive with the antigen to be measured is not particularly limited as long as it is an antigen competitive regarding binding to an antibody that binds to the antigen to be measured. The antigen competitive with the antigen to be measured may be the same antigen as the antigen to be measured, or may be a different antigen. In cases where the competitive antigen is an antigen different from the antigen to be measured, the competitive antigen is preferably an antigen having the same epitope as the epitope recognized by the antibody that binds to the antigen to be measured.

The antifoaming agent used in the present invention is not particularly limited as long as it is an antifoaming agent capable of eliminating air bubbles generated during the dissolution while maintaining the spatial structure of the antibody or antigen. For example, an emulsion-type water-soluble silicone-based antifoaming agent or a self-emulsifying water-soluble silicone-based antifoaming agent may be used. Preferred examples of the emulsion-type water-soluble silicone-based antifoaming agent include KM-7750 (Shin-Etsu Chemical Co., Ltd.), KM-71 (Shin-Etsu Chemical Co., Ltd.), KM-75 (Shin-Etsu Chemical Co., Ltd.), and Antifoam SI (Wako Pure Chemical Industries, Ltd.), and preferred examples of the self-emulsifying water-soluble silicone-based antifoaming agent include KS-538 (Shin-Etsu Chemical Co., Ltd.). Among these, KM-7750 (Shin-Etsu Chemical Co., Ltd.) and Antifoam SI (Wako Pure Chemical Industries, Ltd.) are more preferred. One type of antifoaming agent may be added, or two or more types of antifoaming agents may be added. For example, only an emulsion-type water-soluble silicone-based antifoaming agent may be used; only a self-emulsifying water-soluble silicone-based antifoaming agent may be used; or both may be combined. Regarding the lower limit of the concentration range of the antifoaming agent in the solution before lyophilization, the concentration is preferably not less than 0.0001%, more preferably not less than 0.0005%, still more preferably not less than 0.001% (v/v). Regarding the upper limit, the concentration is preferably not more than 0.1%, more preferably not more than 0.05%, still more preferably not more than 0.01% (v/v). Specifically, the concentration range of the antifoaming agent in the solution before lyophilization is preferably 0.0001 to 0.1% (v/v), more preferably 0.0005 to 0.05% (v/v), especially preferably 0.001 to 0.01% (v/v). For example, the concentration is 0.005% (v/v).

Examples of the solution at this time include a buffer solution and water; and a solution prepared by adding a sugar, salt, protein, or surfactant in a buffer solution or water. Examples of the sugar that may be used include sucrose, mannitol, trehalose, and inositol. Examples of the protein that may be used include bovine serum albumin, collagen peptide, and skim milk. Examples of the surfactant that may be used include anionic surfactants, cationic surfactants, amphoteric surfactants, and nonionic surfactants. Examples of the buffer solution that may be used include Tris, MOPSO, MOPS, and MES, and examples of the salt that may be used include sodium chloride, potassium chloride, magnesium chloride, and zinc chloride. In addition, when necessary, reagent components other than these may be allowed to coexist during the lyophilization.

In the present invention, it is essential that the lyophilized reagent be a lyophilized reagent in which an antifoaming agent is allowed to coexist with a solid-phase antibody or a solid-phase antigen. Further, a lyophilized reagent in which an antifoaming agent is allowed to coexist with a labeled antibody or a labeled antigen may be included.

In the production of the lyophilized reagent of the present invention, a solid-phase antibody or a solid-phase antigen in a solution may be precipitated, and then the solution may be frozen and lyophilized. However, taking the dispersibility after the redissolution into account, freezing and lyophilization are preferably carried out immediately after dispersing the suspension of the solid-phase antibody or the solid-phase antigen, that is, in a state in which the solid-phase antibody or the solid-phase antigen is dispersed.

In this manner, the immunoreaction reagent of the present invention in a lyophilized state can be produced.

As described above, the kit of the present invention comprises: a lyophilized reagent in which an antifoaming agent is allowed to coexist with a solid-phase antibody or a solid-phase antigen; and a reagent containing a labeled antibody or a labeled antigen. At this time, the reagent containing a labeled antibody or a labeled antigen may further contain an antifoaming agent, and may be lyophilized.

### [ADVANTAGEOUS EFFECTS OF INVENTION]

According to the present invention, an immunoreaction reagent in a lyophilized state that shows favorable measurement reproducibility and that is used for in vitro diagnostic agents and the like, and a kit of the reagent, can be obtained. For example, since the immunoassay reagent obtained by the present invention and the kit thereof are capable of accurate measurement, the present invention can also realize highly accurate measurement.

### BRIEF DESCRIPTION OF DRAWINGS

FIG. 1 is a diagram illustrating results of measurement with the reagent in a lyophilized state prepared in Example 1.
FIG. 2 is a diagram illustrating results of measurement with the reagent in a lyophilized state prepared in Comparative Example 1.
FIG. 3 is a diagram illustrating results of measurement with the reagent in a lyophilized state prepared in Example 2.
FIG. 4 is a diagram illustrating results of measurement with the reagent in a lyophilized state prepared in Comparative Example 2.
FIG. 5 is a diagram illustrating conditions of the reagents in a lyophilized state prepared in Example 1 and Comparative Example 1, as observed immediately after dissolution.
FIG. 6 is a diagram illustrating conditions of the reagents in a lyophilized state prepared in Example 1 and Comparative Example 1, as observed 35 seconds after dissolution.
FIG. 7 is a diagram illustrating results of measurement with the reagent in a lyophilized state prepared in Example 4.
FIG. 8 is a diagram illustrating results of measurement with the reagent in a lyophilized state prepared in Comparative Example 3.

### DESCRIPTION OF EMBODIMENTS EXAMPLES

The present invention is described below in more detail by way of Examples. However, the present invention is not limited to Examples.

Using a fully automated chemiluminescent enzyme immunoassay device (AIA-CL2400, manufactured by Tosoh Corporation) as the immunoassay device, and an immunoreaction reagent for the device as the reagent for immunoassay, each measurement was performed by the one-step delay method or the one-step competitive method. Each immunoreaction reagent was prepared as described below.

### (Example 1)

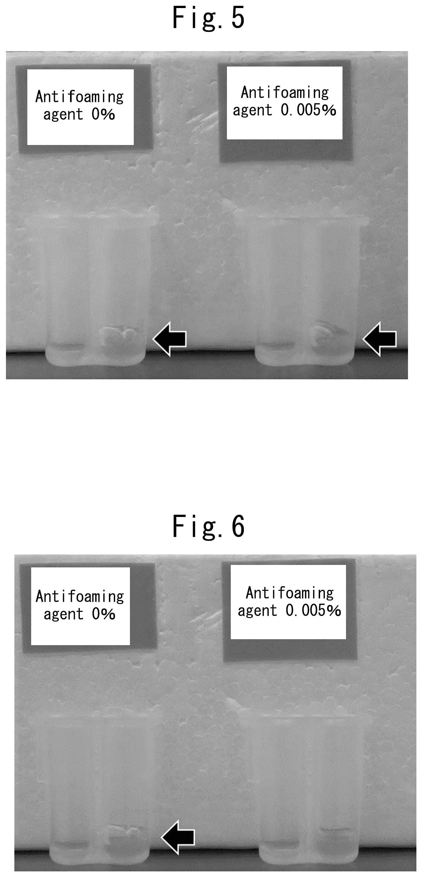

Goat anti-rabbit antibody-immobilized magnetic microparticles were added to Tris buffer (placed in one of the wells of a container having two wells (2-well container) illustrated in FIG. 5) containing a rabbit anti-aldosterone antibody, trehalose, mannitol, potassium chloride, magnesium chloride, zinc chloride, sodium heparin, sodium caseinate, ProClin 950, bovine serum albumin, and collagen peptide, and a silicone-based emulsion-type antifoaming agent (KM-7750 (Shin-Etsu Chemical Co., Ltd.)) was further added to the resulting mixture to a concentration of 0.005% (v/v). The resulting suspension was stirred to completely disperse the magnetic microparticles, and then immediately frozen, followed by performing lyophilization. Subsequently, alkaline phosphatase-labeled aldosterone was added to Tris buffer (placed in the other well of the above-described 2-well container) containing trehalose, collagen peptide, magnesium chloride, zinc chloride, and ProClin 300, and a silicone-based emulsion-type antifoaming agent (KM-7750 (Shin-Etsu Chemical Co., Ltd.)) was further added to the resulting mixture to a concentration of 0.005%(v/v), followed by performing lyophilization.

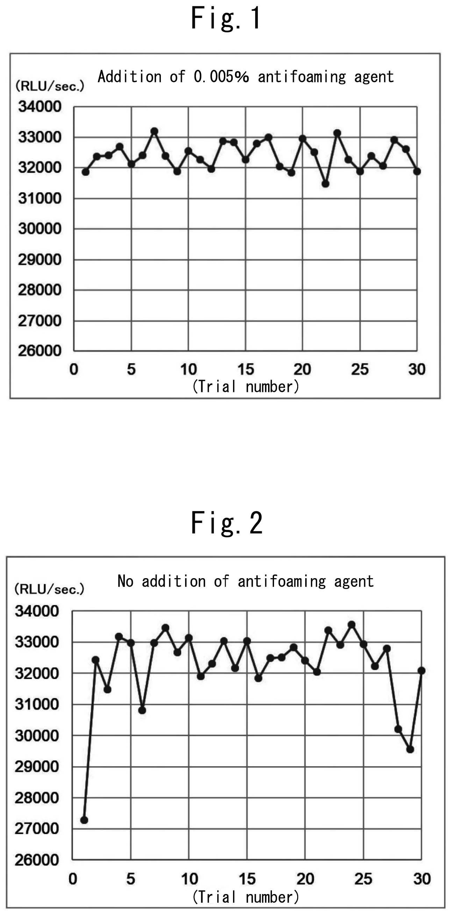

Subsequently, using the thus prepared reagents, an EDTA plasma sample was subjected to measurement by the one-step delay method using the automated immunoassay device, and the luminescence intensity of a chemiluminescent substrate as the substrate of alkaline phosphatase was determined. The EDTA plasma sample was subjected to measurement 30 times, and the resulting average value was taken as the measured value. Further, measurement reproducibility was calculated based on the measured value. The average value of the luminescence intensity was 32392 (RLU/sec.), and the reproducibility (CV: coefficient of variation) was favorable (1.4%). The results are shown in Table 1 and FIG. 1.

**[Table 1]**

| | Example 1 | Comparative Example 1 |
|---|---|---|
| Luminescence intensity (RLU/sec.) | 32392 | 32224 |
| Reproducibility (%) | 1.4 | 4.1 |

### (Comparative Example 1)

Goat anti-rabbit antibody-immobilized magnetic microparticles were added to Tris buffer (placed in one well of a 2-well container illustrated in FIG. 5) containing a rabbit anti-aldosterone antibody, trehalose, mannitol, potassium chloride, magnesium chloride, zinc chloride, sodium heparin, sodium caseinate, ProClin 950, bovine serum albumin, and collagen peptide. The resulting suspension was stirred to completely disperse the magnetic microparticles, and then immediately frozen, followed by performing lyophilization (without addition of an antifoaming agent). Subsequently, alkaline phosphatase-labeled aldosterone was added to Tris buffer (placed in the other well of the above-described 2-well container) containing trehalose, collagen peptide, magnesium chloride, zinc chloride, and ProClin 300, and a silicone-based emulsion-type antifoaming agent (KM-7750 (Shin-Etsu Chemical Co., Ltd.)) was further added to the resulting mixture to a concentration of 0.005%(v/v), followed by performing lyophilization.

Subsequently, using the thus prepared reagents, a test was carried out in the same manner as in Example 1. The average value of the luminescence intensity was 32224 (RLU/sec.), and the resulting reproducibility was inferior (4.1%) to that in Example 1. The results are shown in Table 1 and FIG. 2.

### (Example 2)

Mouse anti-FITC monoclonal antibody-immobilized magnetic microparticles were added to Tris buffer (placed in one well of a 2-well container illustrated in FIG. 5) containing an FITC-labeled S-adenosyl-L-homocysteine BSA conjugate, trehalose, mannitol, dextran, magnesium chloride, zinc chloride, ProClin 950, gentamicin sulfate, collagen peptide, dissociation inhibitor HBR-1, and ultra-low activity alkaline phosphatase. The resulting suspension was stirred to completely disperse the magnetic microparticles, and then immediately frozen, followed by performing lyophilization. Further, an alkaline phosphatase-labeled mouse anti-S-adenosyl-L-homocysteine monoclonal antibody was added to Tris buffer containing trehalose, mannitol, magnesium chloride, zinc chloride, ProClin 950, gentamicin sulfate, and collagen peptide, and a silicone-based emulsion-type antifoaming agent (KM-7750 (Shin-Etsu Chemical Co., Ltd.)) was further added thereto to a concentration of 0.01%(v/v), followed by placing the resulting mixture in the same well of the above-described 2-well container and performing lyophilization.

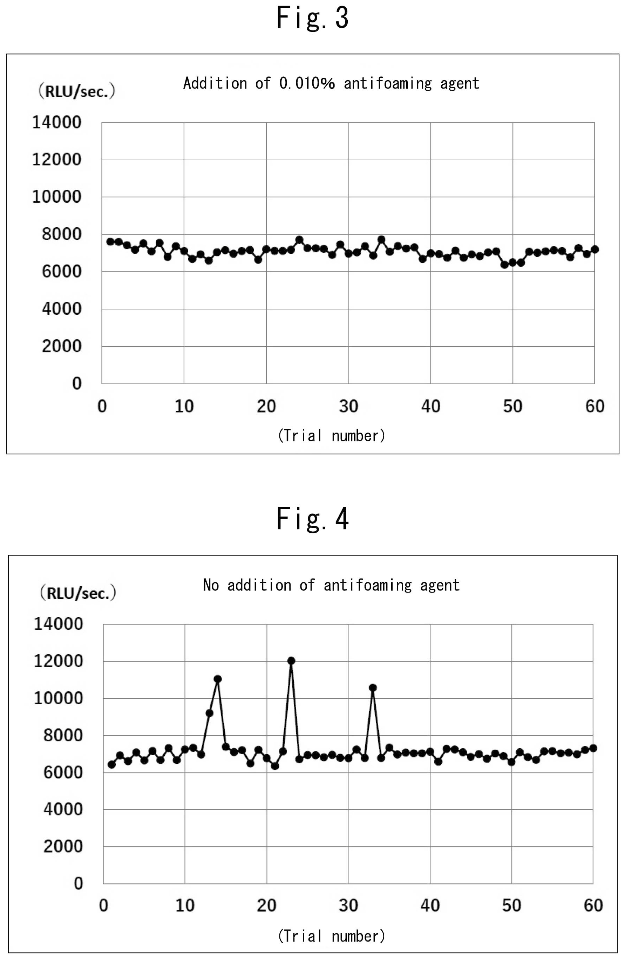

Subsequently, using the thus prepared reagent, a human serum sample was subjected to measurement by the one-step competitive method using the automated immunoassay device, and the luminescence intensity of a chemiluminescent substrate as the substrate for alkaline phosphatase was determined. The human serum sample was subjected to measurement 60 times, and the resulting average value was taken as the measured value. Further, measurement reproducibility was calculated based on the measured value. The average value of the luminescence intensity was 7074 (RLU/sec.), and the reproducibility (CV: coefficient of variation) was favorable (4.2%). The results are shown in Table 2 and FIG. 3.

**[Table 2]**

| | Example 2 | Comparative Example 2 |
|---|---|---|
| Luminescence intensity (RLU/sec.) | 7074 | 7214 |
| Reproducibility (%) | 4.2 | 14.0 |

### (Comparative Example 2)

Mouse anti-FITC monoclonal antibody-immobilized magnetic microparticles were added to Tris buffer (placed in one well of a 2-well container illustrated in FIG. 5) containing an FITC-labeled S-adenosyl-L-homocysteine BSA conjugate, trehalose, mannitol, dextran, magnesium chloride, zinc chloride, ProClin 950, gentamicin sulfate, collagen peptide, dissociation inhibitor HBR-1, and ultra-low activity alkaline phosphatase. The resulting suspension was stirred to completely disperse the magnetic microparticles, and then immediately frozen, followed by performing lyophilization. Further, an alkaline phosphatase-labeled mouse anti-S-adenosyl-L-homocysteine monoclonal antibody was added to Tris buffer containing trehalose, mannitol, magnesium chloride, zinc chloride, ProClin 950, gentamicin sulfate, and collagen peptide, and the resulting mixture was placed in the same well of the above-described 2-well container, followed by performing lyophilization (without addition of an antifoaming agent).

Subsequently, using the thus prepared reagent, a test was carried out in the same manner as in Example 2. The average value of the luminescence intensity was 7214 (RLU/sec.), and the reproducibility was inferior (14.0%) to that in Example 2. The results are shown in Table 2 and FIG. 4.

### (Example 3)

For the 2-well containers containing the reagents in a lyophilized state prepared in Example 1 and Comparative Example 1, a dissolving solution was added to the well containing the magnetic microparticles, to dissolve the reagent. The state of generation of air bubbles was then observed. The reagent in a lyophilized state prepared in Example 1 was defoamed in 35 seconds after the dissolution, while the reagent in a lyophilized state prepared in Comparative Example 1 was not defoamed even after 1 minute had elapsed, and air bubbles were maintained. The state immediately after the dissolution is shown in FIG. 5, and the state 35 seconds after the dissolution is shown in FIG. 6. In FIGs. 5 and 6, the arrows indicate air bubble portions.

In the case of Comparative Example 1, in which no antifoaming agent was included, the air bubbles that had been generated upon the dissolution of the reagent in a lyophilized state did not disappear, and the reproducibility based on 30 times of measurement was 4.1%. In contrast, in the case of Example 1, in which an antifoaming agent was added, the air bubbles that had been generated upon the dissolution of the reagent in a lyophilized state disappeared, and the reproducibility based on 30 times of measurement was greatly improved to 1.4%. This indicates that, by adding the antifoaming agent, the variation caused by air bubbles at the time of the dissolution of the reagent in a lyophilized state was suppressed, resulting in the improvement of the reproducibility of the immunoreaction reagent.

### (Example 4)

A lyophilized reagent was prepared in the same manner as in Example 1 except that silicone-based emulsion-type antifoaming agent Antifoam SI (Wako Pure Chemical Industries, Ltd.) was used as the antifoaming agent.

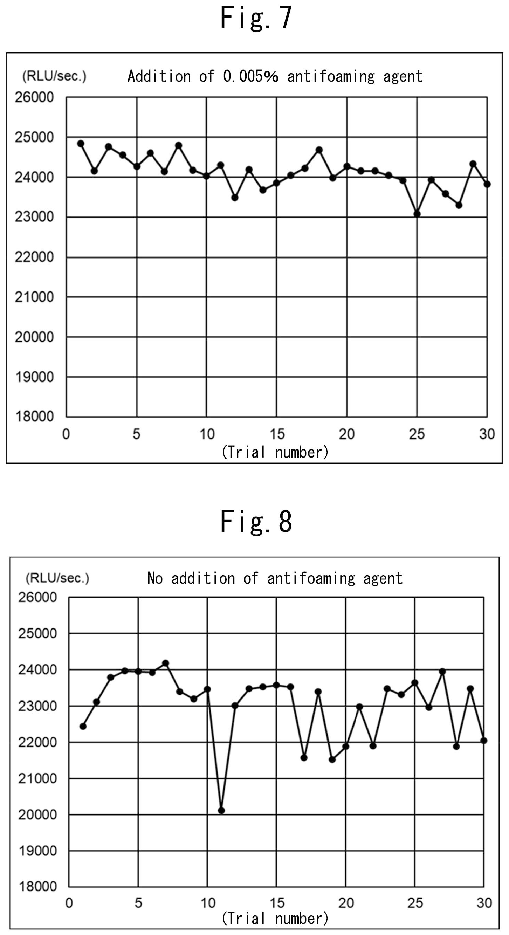

Subsequently, using the thus prepared reagent, measurement was performed 30 times in the same manner as in Example 1, and the resulting average value was taken as the measured value. Further, measurement reproducibility was calculated based on the measured value. The average value of the luminescence intensity was 24115 (RLU/sec.), and the reproducibility (CV: coefficient of variation) was favorable (1.8%). The results are shown in Table 3 and FIG. 7.

**[Table 3]**

| | Example 4 | Comparative Example 3 |
|---|---|---|
| Luminescence intensity (RLU/sec.) | 24115 | 23022 |
| Reproducibility (%) | 1.8 | 4.1 |

### (Comparative Example 3)

A lyophilized reagent was prepared in the same manner as in Comparative Example 1 except that silicone-based emulsion-type antifoaming agent Antifoam SI (Wako Pure Chemical Industries, Ltd.) was used as the antifoaming agent.

Subsequently, using the thus prepared reagent, a test was carried out in the same manner as in Example 4. The average value of the luminescence intensity was 23022 (RLU/sec.), and the reproducibility was inferior (4.1%) to that in Example 4. The results are shown in Table 3 and FIG. 8.

## Claims

1. An immunoreaction reagent comprising:
a lyophilized reagent containing a solid-phase antibody against an antigen to be measured, and an antifoaming agent.

2. An immunoreaction reagent comprising:
a lyophilized reagent containing a solid-phase antigen against an antibody to be measured, and an antifoaming agent.

3. An immunoreaction reagent comprising:
a lyophilized reagent containing a solid-phase antigen that competes with an antigen to be measured for binding to an antibody against the antigen to be measured, and an antifoaming agent.

4. The immunoreaction reagent according to any one of claims 1 to 3, wherein the antifoaming agent is an emulsion-type water-soluble silicone-based antifoaming agent and/or a self-emulsifying water-soluble silicone-based antifoaming agent.

5. A method of producing an immunoreaction reagent, the method comprising: allowing a solid-phase antibody against an antigen to be measured and an antifoaming agent to coexist in a solution; and lyophilizing the solution.

6. A method of producing an immunoreaction reagent, the method comprising: allowing a solid-phase antigen against an antibody to be measured and an antifoaming agent to coexist in a solution; and lyophilizing the solution.

7. A method of producing an immunoreaction reagent, the method comprising:
allowing a solid-phase antigen and an antifoaming agent to coexist in a solution, the solid-phase antigen competing with an antigen to be measured for binding to an antibody against the antigen to be measured; and
lyophilizing the solution.

8. The method of producing an immunoreaction reagent according to any one of claims 5 to 7, wherein the antifoaming agent is an emulsion-type water-soluble silicone-based antifoaming agent and/or a self-emulsifying water-soluble silicone-based antifoaming agent.

9. The method of producing an immunoreaction reagent according to any one of claims 5 to 7, wherein the solid-phase antibody or the solid-phase antigen is dispersed to prepare a suspension, and then the suspension is immediately frozen and lyophilized.

10. An immunoreaction reagent kit comprising:
a lyophilized reagent containing a solid-phase antibody against an antigen to be measured, and an antifoaming agent; and
a reagent containing a labeled antibody capable of binding to the antigen.

11. An immunoreaction reagent kit comprising:
a lyophilized reagent containing a solid-phase antibody against an antigen to be measured, and an antifoaming agent; and
a reagent containing a labeled antigen capable of binding to the solid-phase antibody.

12. An immunoreaction reagent kit comprising:
a lyophilized reagent containing a solid-phase antigen against an antibody to be measured, and an antifoaming agent; and
a reagent containing a labeled antibody capable of binding to the antigen.

13. An immunoreaction reagent kit comprising:
a lyophilized reagent containing a solid-phase antigen and an antifoaming agent, the solid-phase antigen competing with an antigen to be measured for binding to an antibody against the antigen to be measured; and
a reagent containing a labeled antibody capable of binding to the solid-phase antigen and the antigen to be measured.

14. The immunoreaction reagent kit according to any one of claims 10 to 13, wherein the antifoaming agent is an emulsion-type water-soluble silicone-based antifoaming agent and/or a self-emulsifying water-soluble silicone-based antifoaming agent.
